(19) Europäisches Patentamt
European Patent Office
Office européen des brevets

(11) Publication number: 0 138 427 B1

(12) **EUROPEAN PATENT SPECIFICATION**

(45) Date of publication of patent specification: 07.03.90

(21) Application number: 84306464.3

(22) Date of filing: 21.09.84

(51) Int. Cl.$^5$: A 61 L 15/00, B 01 J 20/28, A 61 F 13/15, A 61 F 13/20, A 61 F 5/44, B 01 J 20/30

(54) **Compressed absorbent aggregate.**

(30) Priority: 23.09.83 US 535136

(43) Date of publication of application: 24.04.85 Bulletin 85/17

(45) Publication of the grant of the patent: 07.03.90 Bulletin 90/10

(84) Designated Contracting States: AT BE CH DE FR GB IT LI LU NL SE

(56) References cited:
DE-A-2 649 974
DE-A-3 132 976
US-A-3 670 731
US-A-3 932 322

(73) Proprietor: PERSONAL PRODUCTS COMPANY
Van Liew Avenue
Milltown New Jersey 08850 (US)

(72) Inventor: Pieniak, Heinz Alfred
12 Nathan Drive
North Brunswick New Jersey 08902 (US)

(74) Representative: Jones, Alan John et al
CARPMAELS & RANSFORD
43 Bloomsbury Square
London, WC1A 2RA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 138 427 B1

## Description

The present invention relates to new and improved absorbent products, and more particularly, to new and improved compressed absorbent aggregates of superabsorbent and a filler.

Disposable absorbent products have been known for some time, including such products as disposable diapers, sanitary napkins, wound dressings, bandages, incontinent pads and the like. These products incorporate an absorbent batt which is used to absorb and hold or contain body fluids. Initially, in many of these products, especially diapers and sanitary napkins, the absorbent batt comprised what is termed "wadding" or plies of tissue. The wadding was disposed between an impermeable backing and a permeable facing, and the plies of tissue were used to absorb and hopefully contain the liquid within the product. A diaper which utilizes such an absorbent batt is disclosed in U.S. Reissue Patent No. 26,151.

The wadding type of batt was replaced, for the most part, by an improved absorbent batt which comprises what is termed "fluffed wood pulp fibers". This absorbent batt comprises a layer of individualized wood pulp fibers. The layer having substantial thickness. A diaper which incorporates such a fluffed wood pulp absorbent batt is described in U.S. Patent No. 2,788,003. This diapher had improved absorbency and somewhat better containment than a diaper using a wadding layer. Also, the fluffed wood pulp layer is quite soft, flexible and conformable, and hence, produces an improved diaper over diapers using the wadding as an absorbent layer.

Though the fluffed wood pulp absorbent batts have improved capacity, the capacity is still substantially limited. For instance, in a disposable diaper product the fluid to be absorbed is generally deposited in a localized area within the absorbent batt. Although techniques have been provided to assist the movement of the fluid to other portions of the absorbent batt, the stability of the batt when wet is poor. When the wood pulp fibers become wet, they tend to collapse. Thus limiting the storage area available for the fluid which has been deposited.

U.S. Patent No. 3,017,304 discloses an absorbent product which incorporates in the product a densified paper-like layer. This paper-like layer acts as a wick, i.e., liquid which is placed on the layer tends to move rapidly along the plane of the layer. When incorporated in combination with fluffed wood pulp fibers, the resulting product uses the absorbent capacity of the fluffed wood pulp much more efficiently. Diapers which incorporate this paper-like layer combined with fluffed wood pulp are disclosed and described in U.S. Patent Nos. 3,612,055 and 3,938,522. This concept of combining a wicking or capillary skin or layer with fluffed wood pulp fibers has gained wide acceptance in many absorbent products including disposable diapers and sanitary napkins. Even though these products make much greater use of the capacity of the absorbent batt, they still do not totally contain the absorbed liquid. It is probable that these products will leak before the full capacity of the batt is used for absorption. This is especially true if pressure is placed on the batt while wet. For example, a baby sitting down on a previously wetted diaper will very often cause the batte to leak.

Recently, elastic leg diapers or stretch diapers have been introduced into the marketplate. Though these diapers provide no better absorbent batt than flat diapers or the prior art diapers, they have indicated improved containment of liquid. Such diapers are disclosed and described in U.S. Patent Nos: 3,860,003, 4,050,462 and 4,324,245. Though the containment features are better in the prior art products, the elasticized products fit more tightly permitting less air circulation. Frequently, this can become irritating to the skin and the tighter the elastic or the more close fitting of the diaper, the greater the irritation. This is especially true adjacent the area where the elastic leg portion of the product contacts the wearer.

Another type of liquid body exudate retention product is a container such as a bag, e.g., a colostomy bag, a urine bag or the like, a bedpan or other container-like product. Though such products entrap the liquid exudate, opportunity for spilling the liquid out of the container occurs frequently. Ideally, the liquid when entering the container or absorbent product would be converted to a non-liquid state, or entrapped so that even when pressure is applied there is no leakage or spill-out of liquid.

A number of years ago "superabsorbent materials", i.e., materials which will absorb many times their weight of liquid were developed. Since the development of such materials, people have been trying to incorporate them in absorbent products such as diapers, tampons and sanitary napkins to enhance the absorptive performance of these products. Theoretically, a minimum amount of superabsorbent incorporated in a product would make that product perform as well or better than the prior art products. Perhaps one of the first products to incorporate such a superabsorbent material in a disposable diaper is disclosed in U.S. Patent No. 3,670,731. This patent discloses an absorbent dressing comprising an absorbent layer sandwiched between a permeable facing and an impermeable backing sheet. The absorbent layer contained water insoluble cross-oinked hydrocolloid polymer as the superabsorbent material.

Even though superabsorbent materials have been available for some time, they have not gained wide acceptance in absorbent products such as disposable diapers, sanitary napkins, tampons, colostomy bags, urinary bags, bed pans and kitty litter and the like. A primary reason for this lack of acceptance of the superabsorbents is failure to develop a product capable of economically utilizing the highly increased absorptive capacity of the superabsorbent

material. In order to economically utilize a superabsorbent, the superabsorbent material needs to be placed in contact with the water in its entirety. Thus, the superabsorbent must be available to receive the liquid without impairment by other superabsorbent material which is becoming gel-like. As the superabsorbent material absorbs liquid, it must be allowed to swell. If the superabsorbent is prevented from swelling, it will cease absorbing liquid. Although the void zone is relatively small in the disposable absorbent products and the liquid in a container product resides in a small area, it has not been possible heretofore to place superabsorbent in a relatively small area and have the superabsorbent material function adequately. Over the years a number of techniques have been disclosed in an attempt to provide structures which make efficient use of the superabsorbent material. Such products are disclosed in U.S. Patent Nos. 4,103,062, 4,102,340, and 4,235,237. In addition, methods for incorporating superabsorbent into suitable layers or suitable configurations, which can be placed in an absorbent product, are disclosed in U.S. Patent Nos. 4,186,165, 4,340,057 and 4,364,992. To date, none of these products has met with any substantial commercial success.

Attempts have been made to treat superabsorbent material to improve its ability to accept liquid without impairing the ability of adjacent superabsorbent materials to receive liquid. Such methods are disclosed in U.S. Patent Nos. 3,932,322 and 4,051,086. Presently, none of these methods is being used in commercial products to solve the problem of making the superabsorbent material readily available to rapidly entrap liquid and to continue to absorb liquid.

The present invention provides a new and improved compressed absorbent aggregate which rapidly absorbs liquid and more effectively utilizes a higher portion of the theoretical absorptive capacity of superabsorbent materials. This new aggregate makes use of this capacity even though the liquid to be absorbed is placed in contact with the aggregate in a small region. Furthermore, the new absorbent aggregate may be used in known disposable absorbent products such as disposable diapers and sanitary napkins and containers, such as colostomy bags, etc. Unexpectedly, the new absorbent aggregate will contain liquid in a product such as a disposable diaper without the use of elastic leg members in the product. Surprisingly, the new aggregate will contain absorbed liquid even when pressure is placed upon the product during use. In addition, the new product rapidly absorbs liquid and substantially prevents spill-out from bedpans and other container-like products.

Summary of the invention

The present invention provides a compressed absorbent aggregate comprising a superabsorbent material and a filler. The superabsorbent material is present in an amount from 25 percent to 75 percent by weight of the aggregate, and the filler is present in an amount from 75 percent to 25 percent by weight of the aggregate. The filler and the superabsorbent are blended to become a substantially homogeneous blend prior to being subjected to compression. The compression applied is sufficient to provide granules or tablets and mixtures thereof. The pressure applied is from 100 to 25,000 psi (0.69 to 172.4 MPa). The filler is selected from the group consisting of fumed silica, fumed alumina, aluminum silicates, resilient fibers, vermiculite, perlite, glass fibers, rock wool, ground glass, and diatomaceous earth and the like and mixtures thereof. The superabsorbent material is generally in the form of powder, fibers, or small granules prior to blending.

The compressed absorbent aggregate is readily combined with a fibrous batt to place the aggregate in disposable products, such as disposable diapers, sanitary napkins, and tampons, and is readily utilizable in a container-like product.

The compressed absorbent aggregate of the present invention absorbs a large single void or a continuous stream of liquid many times its weight. Furthermore, the aggregate continues to accept and hold liquid in a quantity approaching its theoretical capacity. In fact, the compressed aggregate has sufficient attraction capability to draw liquid from the paper-like densified layer of a wood pulp fiber batt and from embossed lines placed in the wood pulp fiber batt.

Brief description of the drawings

Figure 1 is a perspective view illustrating the embodiment of the present invention;

Figures 2A, 2B, 2C, 2D, 2E and 2F depict the acceptance of liquids by one embodiment of the present invention over a period of time;

Figure 3 is a perspective view of another embodiment of the present invention;

Figure 3A is a cross-sectional view through lines 3A—3A of Figure 3;

Figure 4 is a perspective view illustrating still another embodiment of the present invention;

Figure 5 is a perspective view of a further embodiment of the present invention;

Figure 6 is a perspective view of a still further embodiment of the present invention;

Figure 7 is a perspective view of the final product of Figure 6;

Figure 8 is a perspective view of another embodiment of the present invention; and

Figure 9 is a perspective view of still another embodiment of the present invention.

Detailed description of the invention

Referring now to the drawings, Figure 1 represents a perspective view of an aggregate of the present invention. The aggregate is a tablet 10 which contains superabsorbent in an amount of 50 percent and a filler in an amount of 50 percent. The filler and the superabsorbent are dry blended and then subjected to a standard tableting operation. Figure 2 is a series of drawings showing the tablet of Figure 1 being placed in water. In Figure 2A, a flat dish 20 has a predetermined amount of

water 22 placed in it, and the tablet 10 is then placed in the dish. In Figure 2B, swelling has started to take place after one second and the tablet 10 begins to lose its identity as a tablet. In Figure 2C, after three more seconds the swelling has substantially increased and the entire tablet has lots its identity and shape as a tablet. In Figure 2D after two additional seconds the tablet 10 has swelled and begun to topple to one side as the superabsorbent is swelling and functioning to the point that each superabsorbent particle is itself swelling and becoming gelatinous. In Figure 2F, after a total of 10 seconds have elapsed, the tablet 10 has now toppled and is primarily a gelatinous mass. Almost all of the water 22 has been absorbed. In Figure 2F, all of the water has been absorbed and what was a tablet is now a gelatinous mass.

Referring now to Figure 3, a sanitary napkin 30 is depicted. A moisture-pervious overwrap 36, such as a nonwoven fabric, provides the exterior surface for the sanitary napkin. Three of the superabsorbent tablets 32 are placed in the sanitary napkin as depicted in Figure 3A. Figure 3A is taken along lines 3A—3A of Figure 3. In Figure 3A, the moisture-pervious overwrap 36A overwraps a liquid barrier 35A, the tablet 32A is in a recessed portion below the liquid barrier 35A. Surrounding the tablet 32A is wood pulp fibre fluff 34A. On the side of the fluff batt opposite the liquid barrier is the moisture-permeable overwrap 36A which permits ingress of body fluid. Thus, as the fluid in the central portion of the sanitary napkin comes into contact with the superabsorbent tablet, the tablet draws the fluid from the absorbent batt and absorbs the liquid, swells and loses its identity as a tablet.

Figure 4 depicts a urinary pad 40 which has a moisture-permeable top surface wrap 42 and a liquid barrier 46 which comes around and is heat bonded to the moisture-permeable surface but leaving an opening as depicted by the dotted lines. An absorbent aggregate 44 in granular form is placed in the interior of the urinary pad in the front portion thereof amongst the wood pulp fibers in the fibrous batt 48. The compressed absorbent aggregate is composed of 60 percent superabsorbent powder blended with 40 percent by weight fumed silica. The blend is then subjected to standard granulating techniques using compression.

In Figure 5, a disposable diaper 50 is depicted. This diaper has a moisture-permeable facing 52, a moisture-impermeable backing 54 and elastic 56 placed in the side margins so as to form elastic legs. In the fibrous batt lying between the facing 52 and the backing 54 are compressed absorbent aggregates 58 in the form of tablets scattered throughout the fibrous batt. To secure the diaper about the waist of the wearer, tape tabs 57 are provided. The diaper product 50 readily accepts liquid through the moisture-impermeable facing 52 primarily in the region wherein the tablets 58 are shown. When the urine touches the tablets, the tablets rapidly absorb it and go through the stages depicted in Figures 2A—2F and become a soft gel. The gel remains beneath the moisture-permeable facing amongst the wood pulp fibers in the absorbent batt of the diaper and prevents the urine from coming back through the facing, thus, keeping the infant very dry.

Figure 6 depicts a blank 60 for manufacturing a tampon consisting of a fibrous batt having granular aggregates 62 lightly pressed into one surface of the fibrous batt. This surface is the surface that is rolled into the interior of the tampon. Figure 7 depicts the tampon 70 in its final form.

Figure 8 illustrates a urinary bag 80 containing at the bottom portion thereof granular aggregates 82.

Figure 9 depicts a typical colostomy bag 90 containing granular aggregates 92.

The aggregates of the present invention comprise at least two components, the first of which is a superabsorbent material. This superabsorbent material is generally a water-insoluble but water-swellable polymeric substance capable of absorbing water in an amount which is at least 10 times the weight of the substance in its dry form. The superabsorbent material is in the form of particles which may be in the shape of fibers, spheres, bits of film, globules, powder or the like.

One suitable form of superabsorbent material may be described chemically as having a backbone of natural or synthetic polymers with hydrophilic groups or polymers containing hydrophilic groups being chemically bonded to the backbone or in intimate admixture therewith. Included in this class of materials are such modified natural and regenerated polymers as polysaccharides including, for example, cellulose and starch and regenerated cellulose which are modified by being carboxylate, phosphonoalkylated, sulphoalkylated or phosphorylated to render them highly hydrophilic. Such modified polymers may also be cross-linked to improve their water-insolubility.

These same polysaccharides may also serve, for example, as the backbone onto which other polymer moities may be bonded by graft copolymerization techniques. Such grafted polysaccharides and their method of manufacture are described in U.S. Patent No. 4,105,033 to Chatterjee et al. and may be described as polysaccharide chains having grafted thereon a hydrophilic chain of the general formula

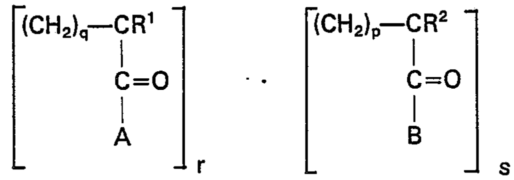

wherein A and B are selected from the group consisting of —$OR^3$, —O (alkali metal), —$OHNH_3$, —$NH_2$, wherein $R^1$, $R^2$ and $R^3$ are selected from the group consisting of hydrogen and alkyl having 1 to 4 or more carbon atoms, wherein r is an integer having a value of 0 to 5000 or more, s is

an integer having a value of 0 to 5000 or more, r plus s is at least 500, p is an integer having a value of zero or 1 and q is an integer having a value of 1 to 4. The preferred hydrophilic chains are hydrolyzed polyacrylonitrile chains and copolymers of polyacrylamide and polysodium acrylate.

In addition to modified natural and regenerated polymers, the hydrocolloid particle component may comprise wholly synthetic hydrophilic particles. Examples of those now known in the art are polyacrylonitrile fibers which may be modified by grafting moities thereon such as polyvinyl alcohol chains, polyvinyl alcohol itself, hydrophilic polyurethane, poly(alkyl phosphonates), partially hydrolyzed polyacrylamides (e.g., poly(N-N-dimethyl acrylamide) sulfonated polystyrene, or a class of poly(alkylene oxide). These highly hydrophilic synthetic polymers may be modified by other chemical treatments such as cross-linking or hydrolysis. Further examples known in the art are the non-ionic hydrophilic polymers such as polyoxyethylene, polyoxypropylene and mixtures thereof which have been suitably cross-linked, either chemically or by irradiation. Still another more recent type is a derivative of isobutylene-maleic anhydride copolymer.

Hydrophilic polymers formed from watersoluble acrylate monomers, such as sodium, potassium, ammonium (or combinations of cations), acrylate, may be placed on the absorbing layer by spraying or otherwise placing a solution thereon followed by polymerization and cross-linking, for example, by irradiation.

Other suitable superabsorbent includes naturally occurring materials such as gums, and the like. Guar gum acacia gum and locust bean gum are examples of suitable gums.

The filler is a non-disintegrating substance selected from the group consisting of fumed silica, fumed alumina, aluminum silicates, fibrous silicates, resilient fibers, vermiculite, perlite, diatomaceous earth, ground glass, glass fibers and the like. The resilient fibers are polyester fibers, thermomechanical pulp, acrylic fibers, extruded silicates, alpha cellulose fibers, and the like. In preparation of the aggregate of the present invention, the filler and the superabsorbent material are blended sufficiently to obtain a substantially homogeneous mixture. The mixture is then formed into granules or tablets as desired. When forming the tablets, the mixture is subjected to compression from 100 psi to 25,000 psi (0.69 to 172.4 MPa). If the superabsorbent material and the filler are of substantially different particle sizes, it may be desirable to add moisture up to 20 percent.

When forming the granular form of the aggregates of the present invention, either wet or dry granulation procedures may be used. In the granulation procedures, compression in the amount of at least 100 psi and preferably 2000 psi (0.69 to 13.8 MPa) is used.

Superabsorbent in the amount of up to 75 percent may be used in the aggregate. If the superabsorbent is in a powder form, it may be desirable and more economical to use a smaller portion of superabsorbent and a larger portion of filler. In other words, when the superabsorbent is a powder, filler may be used in an amount up to 75 percent by weight of the aggregate.

It is theorized that the outstanding result achieved by the present invention is based on a number of happenings. Whether superabsorbent be in the form of particles, fibers, globules, or powder and the like, it is known that the surface area of each particle must come in contact with the liquid in order for the superabsorbent particle to function. When superabsorbent particles are placed in close contact, such as in a layer or pile, and liquid comes in contact with the layer or pile, only those particles on the exterior surface will be able to absorb any liquid, and those particles will absorb liquid only on the exposed side. The side of the layer or pile exposed to the liquid becomes gelatinous and restricts the entry of any more liquid to other parts of the layer or pile. This phenomenon is known in the art as "gel blocking". In order to utilize superabsorbent effectively, it is necessary to disrupt the gel blocking tendency of superabsorbent. In addition, it has been observed that sufficient capillary pressure is needed to continually transport liquid from one particle of superabsorbent to another as well as from one side of a particle to another. It has been discovered that the fillers disrupt the gel blocking of superabsorbents and the pressure applied to make tablets or granules supplies the initial capillary pressure required to transport liquid from the portion contacted by the liquid to another portion.

What appears to be only a small difference in capillary pressure is all that is required for one particle (superabsorbent particle or inert filler particle) to attract and drain liquid from an adjacent particle. The force causing a liquid to enter a cylindrical capillary is expressed by the equation:

$$P=\frac{2\gamma \cos \theta}{r}$$

wherein the force is represented by the capillary pressure and

P is the capillary pressure

$\gamma$ is the surface tension of the liquid

$\theta$ is the liquid-particle contact angle, and

r is the capillary radius.

With a given liquid, the pressure, (capillary force) increases with the cosine of the liquid-fiber contact angle (reaching a maximum where the angle is zero) and also increases with narrower capillary radii so that narrower capillaries will draw liquid from wider ones.

When the aggregate is formed under pressure it is quite possible that the interfaces of the superabsorbent change shape so that interlocking with the filler takes place. The interlocking of

the superabsorbent and filler may be similar to that of jigsaw puzzle pieces. When the superabsorbent is contacted by liquid, it swells resulting in a change of shape which may release it from any interlock or at least the swelling changes the form of attachment of the superabsorbent. As a result, gel blocking is disrupted and a channeling system to permit ingress of liquid toward the interior of the tablet or granule is provided.

The filler preferably is non-swelling, non-disintegrating and hydrophilic. Thus, a low contact angle is provided and the filler readily attracts liquid. The filler also should be non-coalescing in the presence of liquid, and the bonds created by compression should be easily released in the presence of liquid. The superabsorbent should be able to push the filler particles away as it swells while absorbing liquid, otherwise the superabsorbent would be constrained, preventing the continued absorption of liquid.

The tablets or granules of the present invention may be placed in products in a number of ways to enhance utilization of the superabsorbent present. For instance, the tablets may be placed in pockets in an absorbent fluff batt, or lightly adhered to the base of a receptacle. The granules can be placed in a liquid-permeable bag to form a packet or scattered throughout a fibrous pulp layer. Both tablets and granules might be used in the same product, for instance, placed in the folds of a transversely folded or corrugated web. There are many other ways in which the compressed aggregates of the present invention may be used.

Examples of methods of preparing the compressed absorbent aggregates of the present invention are as follows. These examples are not intended to be limiting in any way and extensions and modifications thereof without departure from the spirit and scope of the invention will become apparent from these Examples.

The term "liquid" when used in the following examples is a simulated urine solution of 1% sodium chloride and 0.05% xylene Red B. Dye and which has a surface tension of 30—50 dynes/cm.

Example I

Superabsorbent material identified as Permasorb® 10 manufactured and sold by National Starch Company in the amount of 5 grams is admixed with Hisil, fumed silica manufactured and sold by Hercules Powder Company. The fumed silica is present in an amount of 50 percent by weight of the aggregate. The blend is subjected to a tabletting procedure wherein pressure in the amount of about 10,000 psi (68.9 MPa) for 5 seconds is used. The tablets when in finished form weigh less than 0.5 gram each. When liquid comes in contact with the tablet, the tablet proceeds through the stages shown in Figures 2A—2F in approximately 2 minutes absorbing at least about 10 times the weight of the tablet.

Example II

Superabsorbent fibers prepared in accordance with U.S. Patent 4,105,033 in amount of about 50 parts by weight are admixed with fumed silica in an amount of about 50 parts by weight. The mixture is moistened to about 20 percent by weight of water and subjected to a tabletting operation using a compression of about 10,000 psi (68.9 MPa). These tablets having a diameter of 0.5 inch and weighing about 0.4 gram absorb on an average about 10 milliliters of water in a period of time less than about 2 minutes.

Example III

Superabsorbent material identified as Enka®-PSA is a nonwoven material of bicomponent fibers wherein the core of the fiber is polyester and the sheath of the fiber is polyethylene. The nonwoven web is made by air-laying the fibers and then heating the resulting web sufficiently that the polyethylene becomes tacky and adheres the fibers one to another to provide stability to the web. This web is then subjected to saturation by a solution of the monomer sodium acrylic acid. The saturated web is subjected to irradiation sufficient to polymerize the monomer resulting in adherence of polysodium acrylate to the web in the form of globules and film adhered to the fiber junctions of the web. This web containing polysodium acrylate is ground into particulate material and admixed with thermo-mechanical pulp fibers in an amount of about 50 parts of the superabsorbent material and 50 parts of the thermo-mechanical pulp fibers. The mixture is formed into tablets by subjecting it to compression at about 10,000 psi (68.9 MPa). The liquid holding capacity is increased by about 80 percent and the absorption rate of the Enka®-PSA superabsorbent material by about 200 percent.

Example IV

The Enka®-PSA ground material from Example III is admixed with Hisil in an amount of about 50 parts by weight of Enka®-PSA and 50 parts by weight of Hisil. The mixture is subjected to a standard granulation procedure wherein granules are formed, which have an increased absorption rate of at least 200 percent and an increased liquid holding capacity of at least 50 percent.

Example V

Four grams of Solka-Floc (alpha cellulose fibers, manufactured and sold by Brown Company, New York, New York) and four grams of superabsorbent fibers manufactured according to U.S. Patent 4,105,033 are blended until a homogeneous mixture is obtained. The blend is slugged under a pressure of 5000 psia (34.5 MPa) for five seconds into a tablet (slug) having a diameter of 2.25 inches (5.7 cm).

The slug is dry screened by compaction through a screen of 18 mesh and the granules are collected on a screen of 40 mesh. The average size of granules ranges from 0.425 to 1 mm in diameter.

Other methods for preparing the compressed absorbent aggregates of the present invention may be used.

From the foregoing, it will be observed that numerous variations and modifications may be effected without departing from the true spirit and scope of the novel concept of this invention.

**Claims**

1. A compressed absorbent aggregate comprising superabsorbent material in the amount of 25 to 75 parts by weight blended with from 75 parts to 25 parts by weight of a non-swellable, non-disintregating, hydrophilic filler and subjected to pressure in an amount from 100 to 25,000 psi (0.69 to 172.4 MPa).

2. The aggregate of Claim 1 wherein the pressure forms a tablet.

3. The aggregate of Claim 2 wherein the tablet is broken into at least four pieces.

4. The aggregate of Claim 1 wherein the compression forms a slug which provdes granules.

5. The aggregate of any one of Claims 1 to 4 wherein the filler is selected from the group consisting of fumed silica, fumed alumina, aluminum silicates, resilient fibers, vermiculite, perlite, diatomaceous earth, fibrous silicates, glass fibers, ground glass and mixtures thereof.

6. The aggregate of Claim 5 wherein the resilient fibers are selected from the group consisting of polyester fibers, acrylic fibers, extruded silicate fibers, alpha cellulose fibers and thermo-mechanical pulp fibers.

7. A disposable diaper having as the absorbent pad a loosely compacted cellulosic fibrous web containing therein a compressed absorbent aggregate comprising superabsorbent material in the amount of 25 parts to 75 parts by weight blended with from 75 parts by weight to 25 parts by weight of a non-swellable, non-disintegrating, hydrophilic filler and subjected to pressure in the amount from 100 psi to 25,000 psi (0.69 to 172.4 MPa) to form a compressed aggregate, said disposable diaper containing in the absorbent pad at least about 3 compressed aggregates.

8. A disposable diaper as in Claim 7 wherein said compressed aggregates are tablets.

9. A disposable diaper as in Claim 7 wherein said compressed aggregates are granules.

10. A sanitary napkin comprising an absorbent pad partially encompassed by a liquid barrier with a moisture-permeable overwrap, said absorbent pad being a lightly compacted cellulosic fibrous web containing a compressed absorbent aggregate comprising superabsorbent material in the amount of 25 parts to 75 parts by weight blended with from 75 parts to 25 parts by weight of a non-swellable, non-disintegrating, hydrophilic filler and subjected to pressure in the amount from 100 psi to 25,000 psi (0.69 to 172.4 MPa) to form a compressed aggregate, said napkin containing at least about two compressed aggregates in said fibrous web.

11. A sanitary napkin as in Claim 10 wherein said compressed aggregates are tablets.

12. A sanitary napkin as in Claim 10 wherein said compressed aggregates are granules.

13. A tampon comprising a loosely compacted cellulosic fibrous web containing a compressed absorbent aggregate comprising superabsorbent material in an amount of 25 parts to 75 parts by weight blended with from 75 parts to 25 parts by weight of a non-swellable, non-disintegrating, hydrophilic filler and subjected to pressure in the amount of from 100 psi to 25,000 psi (0.69 to 172.4 MPa) to form a compressed aggregate.

14. A tampon as in Claim 13 wherein said compressed aggregate is a tablet.

15. A tampon as in Claim 13 wherein said compressed aggregate is a multiplicity of granules.

16. A colostomy bag containing at least about two compressed aggregates as defined in any one of Claims 1 to 6.

17. A urinary bag containing at least about two compressed aggregates as defined in any one of Claims 1 to 6.

18. A method for forming a compressed absorbent aggregate comprising admixing superabsorbent material in the amount of 25 parts to 75 parts by weight with from 75 parts to 25 parts by weight of a non-swellable, non-disintegrating, hydrophilic filler and subjecting the resulting admixture to pressure in the amount from 100 psi to 25,000 psi (0.69 to 172.4 MPa) for a period of at least about five seconds.

**Patentansprüche**

1. Ein zusammengepreßtes absorbierendes Aggregat, welches ein superabsorbierendes Material in einer Menge von 25 Gew.-Teilen bis 75 Gew.-Teilen, vermischt mit 75 Gew.-Teilen bis 25 Gew.-Teilen eines nicht-quellbaren, nicht-zerfallenden hydrophilen Füllstoffes enthält, und welches einem Druck in einer Höhe von 100 psi bis 25.000 psi (0,69 bis 172.4 MPa) unterworfen worden ist.

2. Das Aggregat nach Anspruch 1, wobei durch den Druck eine Tablette gebildet wird.

3. Das Aggregat nach Anspruch 2, wobei die Tablette auf wenigstens vier Stück zerbrochen wird.

4. Das Aggregat nach Anspruch 1, wobei durch die Kompression ein Preßkörper gebildet wird, aus dem dann ein Granulat erzeugt wird.

5. Das Aggregat nach einem der Ansprüche 1 bis 4, wobei der Füllstoff aus der Gruppe ausgewählt ist, die aus pyrogener Kieselsäure, pyrogenem Aluminiumoxid, Aluminiumsilikaten, elastischen Fasern, Vermiculit, Perlit, Diatomeenerde, Fasersilikaten, Glasfasern, gemahlenem Glas und Gemischen davon besteht.

6. Das Aggregat nach Anspruch 5, wobei die elastischen Fasern aus der Gruppe ausgewählt sind, die aus Polyesterfasern, Acrylfasern, extrudierten Silikatfasern, α-Zellulosefasern und thermomechanischen Zellstoffasern besteht.

7. Eine Wegwerfwindel, welche als absorbierendes Kissen eine locker verdichtete Faserbahn auf Zellulosebasis aufweist, welche Faserbahn ein zusammengepreßtes absorbierendes Aggregat enthält, welches Aggregat seinerseits ein superabsorbierendes Material in einer Menge von 25 Gew.-Teilen bis 75 Gew.-Teilen, vermischt mit 75 Gew.-Teilen bis 25 Gew.-Teilen eines nicht-quellbaren, nicht-zerfallenden hydrophilen Füllstoffes enthält und welches Aggregat zur Bildung eines zusammengepreßten Aggregates einem Druck in einer Höhe von 100 psi bis 25.000 psi (0,69 bis 172,4 MPa) unterworfen worden ist, wobei diese Wegwerfwindel wenigstens etwa drei zusammengepreßte Aggregate in dem absorbierenden Kissen enthält.

8. Eine Wegwerfwindel nach Anspruch 7, worin die zusammengepreßten Aggregate Tabletten sind.

9. Eine Wegwerfwindel nach Anspruch 7, worin due zusammengepreßten Aggregate Körner sind.

10. Eine Damenbinde, welche ein absorbierendes Kissen umfaßt, welches teilweise von einer Flüssigkeitssperre und mit einer feuchtigkeitsdurchlässigen Umhüllung umgeben ist, wobei dieses absorbierende Kissen eine leicht verdichtete Faserbahn auf Zellstoffbasis ist, welche Faserbahn ein zusammengepreßtes absorbierendes Aggregat enthält, welches Aggregat seinerseits ein superabsorbierendes Material in einer Menge von 25 Gew.-Teilen bis 75 Gew.-Teilen, vermischt mit 75 Gew.-Teilen bis 25 Gew.-Teilen eines nicht-quellbaren, nichtzerfallenen hydrophilen Füllstoffes enthält und welches Aggregat zur Bilding eines zusammengepreßten Aggregates einem Druck in einer Höhe von 100 psi bis 25.000 psi (0,69 bis 172,4 MPa) unterworfen worden ist, wobei diese Damenbinde wenigstens etwa zwei zusammengepreßte Aggregate in der Faserbahn enthält.

11. Eine Damenbinde nach Anspruch 10, worin die zusammengepreßten Aggregate Tabletten sind.

12. Eine Damenbinde nach Anspruch 10, worin die zusammengepreßten Aggregate Körner sind.

13. Ein Tampon, welcher eine locker verdichtete Faserbahn auf Zellulosebasis umfaßt, welche Faserbahn ein zusammengepreßtes absorbierendes Aggregat enthält, welches Aggregat seinerseits ein superabsorbierendes Material in einer Menge von 25 Gew.-Teilen bis 75 Gew.-Teilen, vermischt mit 75 Gew.-Teilen bis 25 Gew.-Teilen eines nicht-quellbaren, nicht zerfallenden hydrophilen Füllstoffes enthält, und welches Aggregat zur Bildung eines zusammengepreßten Aggregates einem Druck in einer Höhe von 100 psi bis 25.000 psi (0,69 bis 172,4 MPa) unterworfen worden ist.

14. Ein Tampon nach Anspruch 13, worin das zusammengepreßte Aggregat ein Tablette ist.

15. Ein Tampon nach Anspruch 13, worin das zusammengepreßte Aggregat eine Vielzahl von Körnern ist.

16. Ein Kolostomiebeutel, der wenigstens etwa zwei wie in einem der Ansprüche 1 bis 6 definierte zusammengepreßte Aggregate enthält.

17. Ein Harnaufnahmebeutel, der wenigstens etwa zwei wie in einem der Ansprüche 1 bis 6 definierte zusammengepreßte Aggregate enthält.

18. Ein Verfahren zur Bildung eines zusammengepreßten absorbierenden Aggregates, welches das Vermischen von superabsorbierendem Material in einer Menge von 25 Gew.-Teilen bis 75 Gew.-Teilin mit 75 Gew.-Teilen bis 25 Gew.-Teilen eines nicht-quellbaren, nichtzerfallenden hydrophilen Füllstoffes und das Einwirkenlassen eines Drucks in einer Höhe von 100 psi bis 25.000 psi (0,69 bis 172,4 Mpa) während eines Zeitraumes von wenigstens etwa fünf Sekunden auf das erhaltene Gemisch umfaßt.

**Revendications**

1. Agrégat absorbant comprimé comprenant une matière superabsorbante à raison de 25 à 75 parties en poids mélangée avec 75 à 25 parties en poids d'une charge hydrophile, non gonflable et non désintégrante et qu'on soumet à une pression comprise entre 0,69 et 172,4 MPa (100 à 25.000 psi).

2. Agrégat selon la revendication 1, dans lequel la pression forme un comprimé.

3. Agrégat selon la revendication 2, dans lequel le comprimé est brisé en quatre morceaux au moins.

4. Agrégat selon la revendication 1, dans lequel la compression forme une briquette qui donne des granules.

5. Agrégat selon l'une quelconque des revendications 1 à 4, dans lequel la charge est choisie parmi la silice fumée, l'alumine fumée, les silicates d'aluminium, des fibres résilientes, la vermiculite, la perlite, la diatamite, les silicates fibreux, les fibres de verre, le verre dépoli et des mélanges de ceux-ci.

6. Agrégat selon la revendication 5, dans lequel les fibres résilientes sont choisies parmi les fibres de polyesters, les fibres acryliques, les fibres de silicates extrudées, les fibres d'alpha-cellulose et les fibres de pâte thermomécanique.

7. Couche jetable comportant à titre de coussinet absorbant, une bande fibreuse cellulosique agglomérée de façon lâche et contenant un agrégat absorbant comprimé, qui comprend une matière superabsorbante à raison de 25 à 75 parties en poids en mélange avec 75 à 25 parties en poids d'une charge hydrophile non gonflable et non désintégrable, soumis à une pression comprise entre 0,69 et 172,4 MPa (100 à 25000 psi) pour former un agrégat comprimé, ladite couche jetable contenant dans le tampon absorbant au moins trois agrégats comprimés environ.

8. Couche jetable selon la revendication 7, dans laquelle lesdits agrégats comprimés sont des comprimés.

9. Couche jetable selon la revendication 7, dans laquelle lesdits agrégats comprimés sont des granules.

10. Serviette hygiènique comprenant un coussinet absorbant partiellement entouré d'une barrière antiliquide entourée d'une enveloppe extérieure perméable à l'humidité, ledit coussinet absorbant étant une bande fibreuse cellulosique légèrement agglomérée contenant un agrégat absorbant comprimé qui comprend une matière superabsorbante à raison de 25 à 75 parties en poids en mélange avec 75 à 25 parties en poids d'une charge hydrophile non gonflable et non désintégrable et soumis à une pression comprise entre 0,69 et 172,4 MPa (100 à 25000 psi) pour former un agrégat comprimé, ladite serviette contenant au moins deux agrégats comprimés environ dans ladite bande fibreuse.

11. Serviette hygiènique selon la revendication 10, dans laquelle lesdits agrégats comprimés sont des comprimés.

12. Serviette hygiènique selon la revendication 10, dans laquelle lesdits agrégats comprimés sont des granules.

13. Tampon périodique comprenant une bande fibreuse cellulosique agglomérée de façon lâche et contenant un agrégat absorbant comprimé qui comprend une matière superabsorbante à raison de 25 à 75 parties en poids en mélange avec 75 à 25 parties en poids d'une charge hydrophile non gonflable et non désintégrable, soumis à une pression à raison de 0,69 à 172,4 MPa (100 à 25.000 psi) pour former un agrégat comprimé.

14. Tampon périodique selon la revendication 13, dans lequel ledit agrégat comprimé est un comprimé.

15. Tampon périodique selon la revendication 13, dans lequel ledit agrégat comprimé est une multiplicité de granules.

16. Poche de colostomie contenant au moins deux agrégats comprimés environ tels que définis dans l'une quelconque des revendications 1 à 6.

17. Poche urinaire contenant au moins deux agrégats comprimés environ tels que définis dans l'une quelconque des revendications 1 à 6.

18. Procédé de formage d'un agrégat absorbant comprimé, qui consiste à mélanger une matière superabsorbante à raison de 25 à 75 parties en poids avec 75 à 25 parties en poids d'une charge hydrophile non gonflable et non désintégrable et à soumettre le mélange résultant à une pression de 0,69 à 172,4 MPa (100 à 25.000 psi) pendant une durée d'au moins 5 secondes environ.

FIG-1

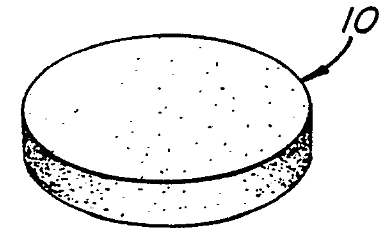


FIG-2A

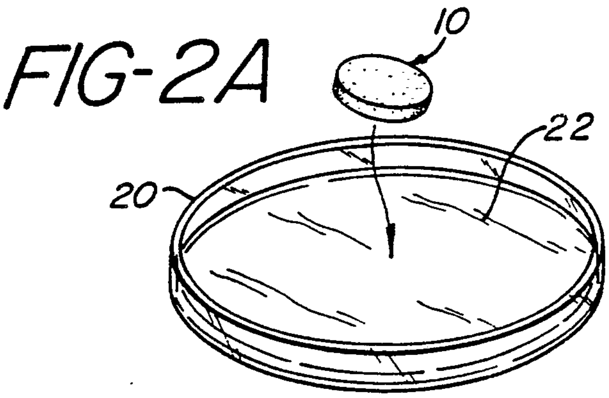


FIG-2B

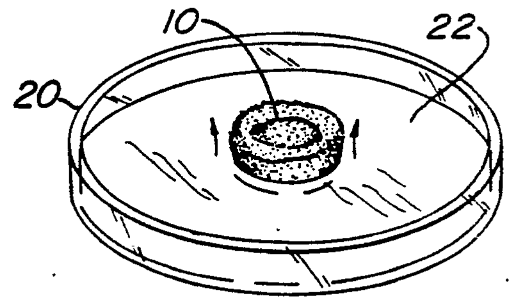


FIG-2C

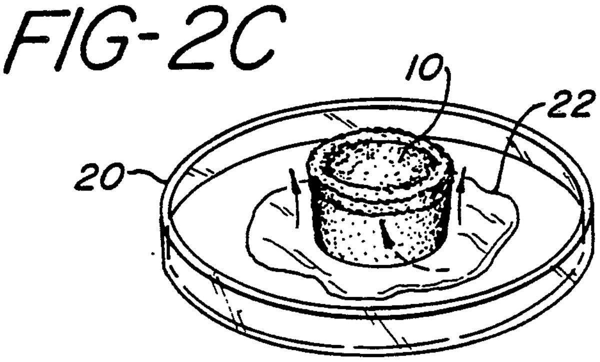


FIG-2D

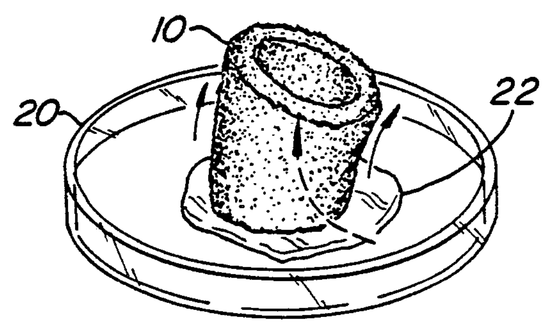


FIG-2E

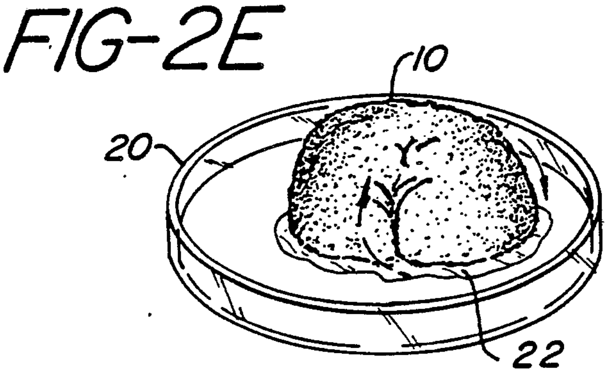


FIG-2F

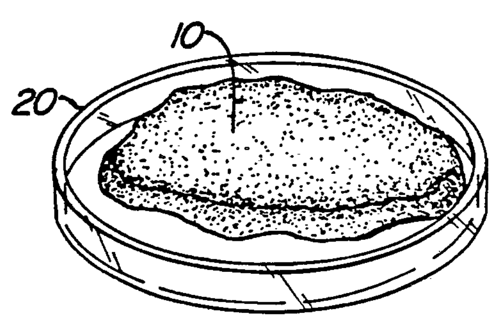

FIG-3

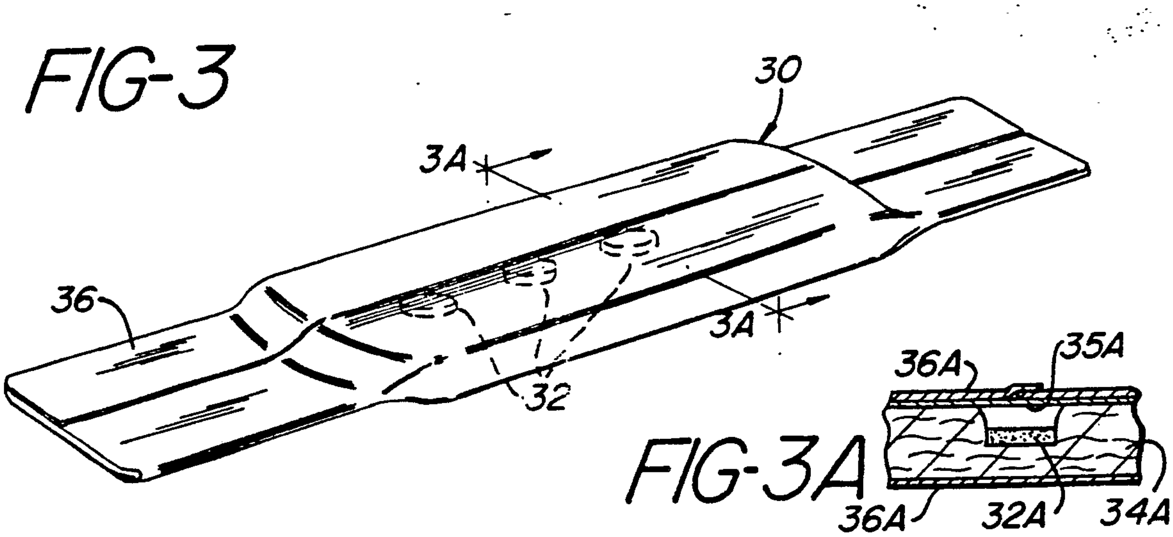


FIG-3A

FIG-4

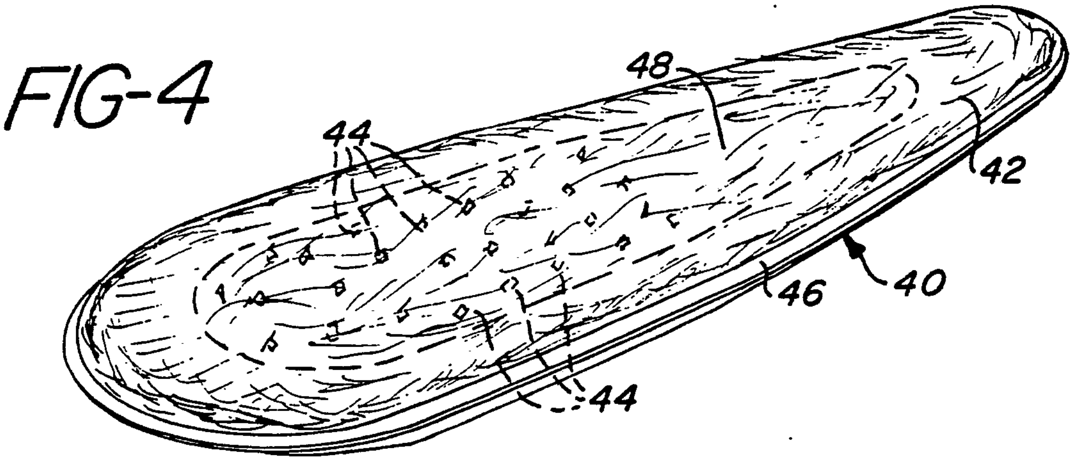


FIG-5

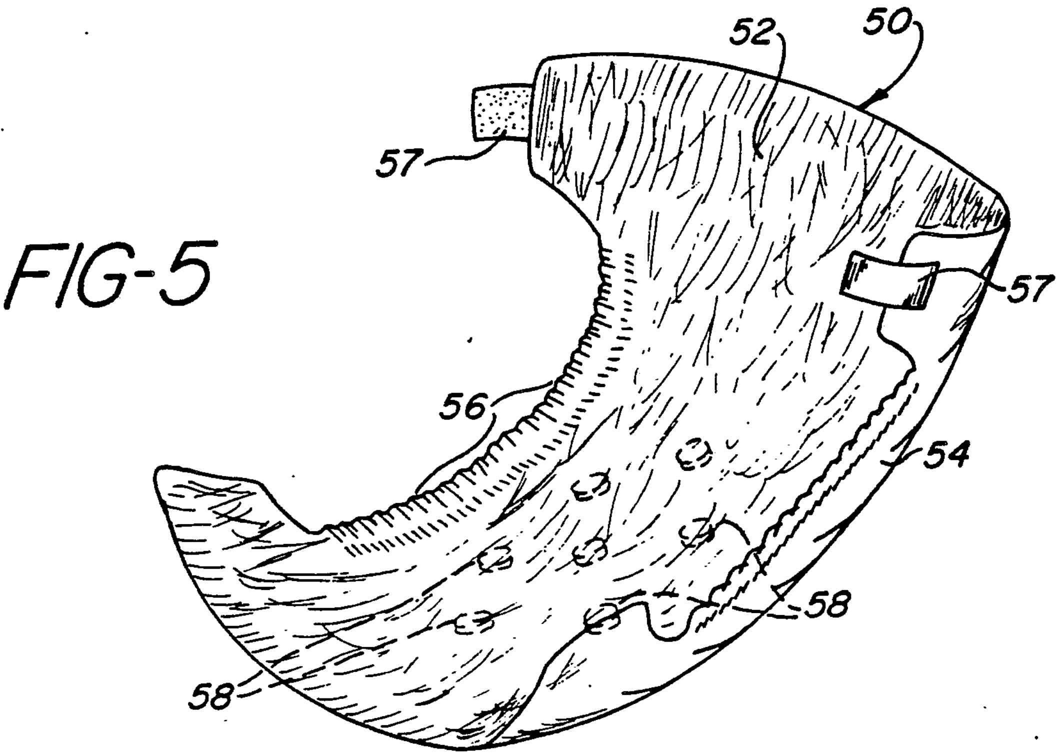

FIG-6

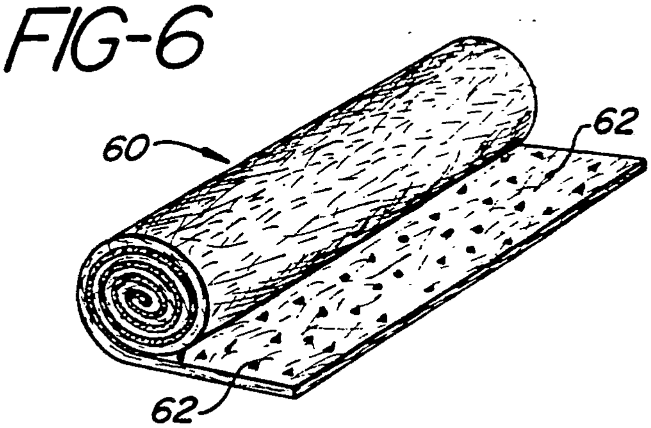


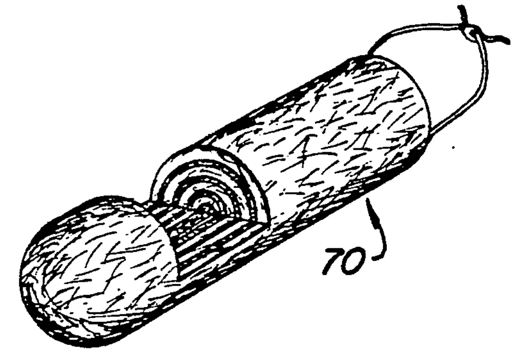


FIG-7

FIG-8

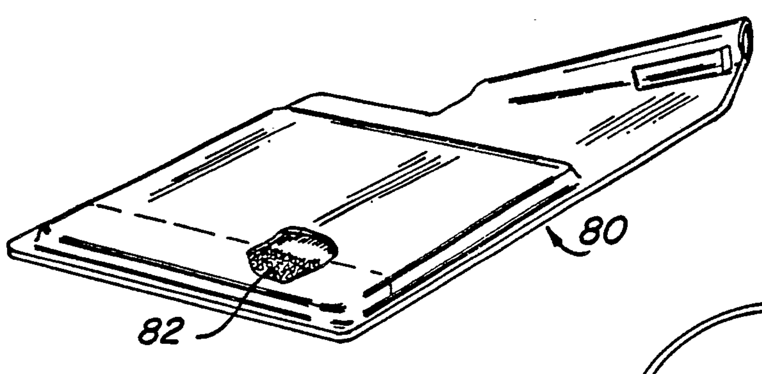


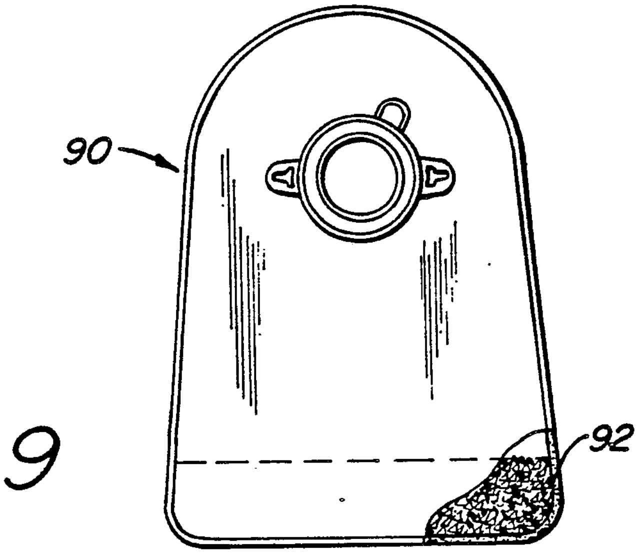


FIG-9